# EUROPEAN PATENT SPECIFICATION

(11) **EP 3 823 532 B1**
(45) Date of publication and mention of the grant of the patent: **04.09.2024**
(21) Application number: 19749457.8
(22) Date of filing: 17.07.2019
(51) Int. Cl.: A61B 6/08, A61B 8/08, A61B 5/00, A61B 5/06, A61B 17/34, A61M 25/01, A61M 5/42, A61B 5/107, A61M 25/06, A61B 17/225, A61M 5/20, A61M 5/158

(54) **CANNULA INSERTION SYSTEM**
KANÜLENEINFÜHRSYSTEM
SYSTÈME D'INSERTION DE CANULE

(30) Priority: 17.07.2018 NL 2021329
(43) Date of publication of application: 26.05.2021
(73) Proprietor: Vitestro Holding B.V., 3526 KS Utrecht (NL)
(72) Inventor: OVERBEEKE, Toon Olaf, 3533 AB Utrecht (NL); JOSEPH, Brian Robert, 2597 EZ 'S-Gravenhage (NL); KARSSEN, Johannes Gerhard Daniël, 3526 KS Utrecht (NL); JONKER, Arris Cornelis, 3526 KS Utrecht (NL)
(74) Representative: EP&C
(86) International application number: PCT/NL2019/050455
(87) International publication number: WO 2020/017965

(56) References cited:
- EP-A1- 2 749 303
- WO-A2-2006/120619
- CN-A- 104 605 865
- CN-A- 106 580 344
- US-A1- 2007 088 346
- US-A1- 2011 166 451
- US-A1- 2017 028 142

## Description

The present invention relates to a cannula insertion system for inserting a cannula into a blood vessel of a human or animal.

US2008/0275396 A1 discloses an automated cannula insertion system for autonomously inserting a cannula into a blood vessel of a human or an animal, the contents of which are herein incorporated by reference in its entirety.

The cannula insertion system of US2008/0275396 A1 comprises an acquisition module that allows for determining at least a location of a blood vessel underneath the skin and is enabled to determine a puncture location that is suitable for inserting a cannula into the blood vessel. The cannula insertion system comprises an actuator for moving and aligning the cannula to a determined position and to autonomously insert the cannula into the blood vessel for multiple purposes, such as blood withdrawal, venous medication and infusions.

In an embodiment of the cannula insertion system of US2008/0275396 A1, an ultrasound transducer is provided to obtain image data that can be used to localize a blood vessel. To properly obtain image data from the human or animal, a so-called coupler medium is needed to transmit ultrasound waves from the ultrasound transducer into the body of the human or animal, and to receive reflected ultrasound waves from the body of the human or animal into the ultrasound transducer. This coupler medium is usually a so-called ultrasound gel which is manually applied to the skin of the human or animal before the ultrasound transducer is placed on the skin.

After the imaging with the ultrasound transducer is finished, the ultrasound transducer and the skin of the human or animal have to be thoroughly cleaned to remove all remaining ultrasound gel. As long as the ultrasound gel is present on the ultrasound transducer and/or the skin of the human or animal, other parts of the cannula insertion system may be contaminated with the ultrasound gel. This is generally undesirable.

WO 2006/120619A2 and US 2017/0028142 discloses an automatic needle insertion apparatus. US2011/0166451 discloses a device having a needle injector pivotally attached to an ultrasound receiver. US 2007/0088346 A1 discloses a method for treating a perforator vein, said method comprising applying ultrasound to the perforator vein and occluding the perforator vein with ultrasound. EP2749303, CN106580344 and CN104605865 disclose devices to introduce a needle into a patient.

An object of the present invention is to provide a cannula insertion system in which a coupler medium may be efficiently used, preferably lacking one or more of the above mentioned drawbacks of the prior art ultrasound gel.

The present invention provides a cannula insertion system as claimed in claim 1.

In accordance with the present invention, the cannula insertion system comprises a coupler liquid application device to apply before and/or during measuring with the contact sensor coupler liquid onto a target area of the skin of the human or animal. The advantage of such coupler liquid application device is that the coupler liquid will only be applied to the skin of the human or animal, for example the arm of the human or animal, after this body part is correctly placed within the cannula insertion system. The coupler liquid application device may be arranged at a suitable position for the application of the coupler liquid. As a result, the chance that the cannula insertion system will be contaminated by coupler medium as a result of for example insertion of an arm into the cannula insertion system or as a result of applying a coupler medium manually after the respective body part has been placed into the cannula insertion system is substantially reduced.

The target area refers to an area of skin which the cannula insertion system examines to find a suitable location for insertion of a cannula into the body. The coupler liquid application device is arranged to apply coupler liquid in at least a part of this target area.

The coupler liquid application device is activated by the cannula insertion system, instead of being operated manually by an operator of the cannula insertion system. Such coupler liquid application device being operated by the cannula insertion system facilitates the autonomous operation of the cannula insertion system, i.e. less interaction of an operator is needed in order to insert a cannula into a body of a human or an animal.

The cannula insertion system may comprise a coupler liquid control device to operate the coupler liquid application device. This coupler liquid control device may be the same control device as the control device arranged to control the positioning system, or a separate control device.

The ultrasound transducer uses ultrasound waves to obtain image data on the basis of which the location of a blood vessel within the body part may be determined.

In a cannula insertion system according to the invention, multiple types of sensors may be provided to obtain data that is representative or relevant for the determination of a blood vessel under the skin of a human or animal.

The sensor signals obtained by the one or more sensors are fed into a processing device. The processing device is arranged to determine the location of a blood vessel suitable for insertion of a cannula.

The coupler liquid application device is a spray device arranged to spray coupler liquid on the target area. Spraying of coupler liquid is a suitable method to efficiently apply coupler liquid in the target area.

In alternative embodiments not covered by the invention, the coupler liquid application device may for example be a drip device that drips coupler liquid on the target area. Preferably, the coupler liquid is applied to the skin without contact between the coupler liquid application device and the skin.

In an embodiment, the coupler liquid is an alcohol based liquid. The advantage of an alcohol based liquid is that the liquid will evaporate after it has been applied to the skin. This means that after the cannula has been inserted into the body of the human or animal, all or at least a substantial part of the coupler liquid will have been evaporated from the skin. This substantially reduces the chance that the cannula insertion system will be contaminated with coupler liquid during removal of the respective body part from the cannula insertion system. Also, the ultrasound transducer and the skin of the human or animal has to be cleaned less thoroughly after insertion of a cannula when compared to the application of ultrasound gel as a coupler medium. The presence of coupler liquid on the skin may also make it more difficult to apply a bandage on the skin after the cannula has been removed from the blood vessel. The use of coupler liquid that relatively quickly evaporates, facilitates the application of a bandage after removal of the cannula.

A further advantage of an alcohol based liquid is that it may act as a disinfectant.

In an embodiment, the coupler liquid is an antiseptic liquid. Since the cannula insertion system is configured to insert a cannula into the body of a human or animal, it is advantageous to provide an antiseptic liquid as a coupler medium, since this antiseptic liquid will be applied on the target area of the skin into which a cannula is to be inserted. The coupler liquid is then not only used as a coupler medium to transmit ultrasound waves between the ultrasound transducer and the body of the human or animal, but also to disinfect the skin of the target area where the cannula is inserted into the body.

In other embodiments, the coupler liquid may be any suitable liquid such as water or sprayable ultrasound gel.

The antiseptic coupler liquid may be used as the only disinfectant to disinfect the target area of the skin, or may be used as an additional disinfectant that is used in addition to disinfecting the target area before the respective body part is introduced into the cannula insertion system.

In an embodiment, the coupler liquid application device comprises a container to hold the coupler liquid and a spray nozzle in fluid communication with the container, wherein the spray nozzle is positioned to spray coupler liquid onto the target area. The coupler liquid application device may be configured as any suitable device to spray coupler liquid onto the target area. The container may be a replaceable container to easily replenish the coupler liquid. The spray nozzle is configured to spray the coupler liquid onto the target area, whereby the coupler liquid is preferably evenly distributed over the target area to create a liquid film on the skin that effectively acts as a coupler medium between the ultrasound transducer and the skin.

Since the spray nozzle of the coupler liquid application device has to be brought in a proper position with respect to the target area in order to spray the coupler liquid efficiently onto the target area, it is advantageous that the spray nozzle is movable by one or more actuators of a spray device positioning system. These one or more actuators may be provided to move the spray device, or at least the spray nozzle thereof, independently of other movable parts of the cannula insertion system, such as the contact sensor and the cannula insertion device.

In another embodiment, the coupler liquid application device, or at least a spray nozzle thereof, is movable by the positioning system of the cannula insertion device. In this embodiment, the positioning system may also be used to support the coupler liquid application device, such that the coupler liquid application device can be positioned in a desired position using the positioning system.

The positioning of the coupler liquid application device may be controlled by a control device. This control device may be a separate control device which is only used to control the position of the coupler liquid application device, but may also be a central control device of the cannula insertion system.

In an embodiment, the coupler liquid application device comprises a pump to apply coupler liquid onto the target area, wherein the pump is operated by the control device. The pump may be any suitable pump. Preferably, the pump is suitable to pump, when desired, coupler liquid to a spray nozzle with sufficient pressure to form a spray jetting from the spray nozzle. The pump is for example a piston pump with an actuator that can be activated by the control device. This control device may be the same control device as the control device controlling the position of the coupler liquid application device, or a part thereof, for example a spray nozzle of the spray device.

In an embodiment, the ultrasound transducer is movable, for example by a ultrasound transducer positioning system, and movements of the ultrasound transducer are controlled by the control device.

In an embodiment, the cannula insertion device, the ultrasound transducer and the coupler liquid application device are arranged in a measurement and cannula insertion unit, wherein the measurement and cannula insertion unit is movable by the positioning system under control of the control device. The measurement and cannula insertion unit supports simultaneously the cannula insertion device, the ultrasound transducer and the coupler liquid application device. A single positioning system may be provided to position the cannula insertion device, the ultrasound transducer and the coupler liquid application in a desired position by respective movement of the measurement and cannula insertion unit. The positioning system may comprise a robot arm.

In an embodiment, the control device and the processing device are integrated in a single processing unit, for example a PC.

In an embodiment, the cannula insertion system is constructed to autonomously insert the cannula into the blood vessel. This means that the cannula insertion system may be configured to carry out, without any interaction of a human operator, the following steps:
applying, using the coupler liquid application device, before and/or during measuring with the contact sensor, coupler liquid onto the target area of the skin of the human or animal,
measuring, using the ultrasound transducer, to provide one or more sensor signals representative for a location of the blood vessel,
determining, using the processing device, a location of a blood vessel on the basis of the one or more sensor signals,
positioning, on the basis of the determined location of the blood vessel, the cannula insertion device in a suitable position to insert the cannula into the blood vessel, and
inserting the cannula into the blood vessel.

In an embodiment, the cannula insertion system may further be configured to carry out the step of determining with one or more sensors a target area. The target area may be determined by determining the location of one or more blood vessels, for example using an NIR (near infrared) imaging device and selecting a target area on the basis of the determined one or more blood vessels.

Applying the coupler liquid comprises spraying the coupler liquid with a spray device.

In an embodiment, the cannula is a needle. The cannula may be any tube like element that is configured to be introduced into a blood vessel. Preferably, the cannula is a needle, in particular a needle suitable to draw blood from the blood vessel.

In an embodiment, the cannula insertion system is constructed to autonomously draw blood from the blood vessel. In other embodiments, the cannula insertion system may be used to insert a cannula into a blood vessel for venous medication or infusions.

The invention also provides a method for determining a location of a blood vessel of a human or animal, using the cannula insertion system of any of the claims 1-16, the method comprising the steps of
applying, using the coupler liquid application device, before and/or during measuring with the contact sensor, coupler liquid onto the target area of the skin of the human or animal, wherein applying the coupler liquid comprises spraying the coupler liquid with the spray device.
   and
measuring with the ultrasound transducer to provide one or more sensor signals representative for a location of the blood vessel,
determining a location of a blood vessel on the basis of the one or more sensor signals.

In an embodiment, the method may comprise the step of determining with one or more sensors a target area.

Further characteristics and advantages of the cannula insertion system of the invention will now be explained by description of an embodiment of the invention, whereby reference is made to the appended drawings, in which:
Figure 1 shows schematically a cannula insertion system according to an embodiment of the invention;
Figure 2 shows a measurement and cannula insertion unit according to an embodiment of the invention; and
Figure 3 shows schematically a spray device according to an embodiment of the invention.

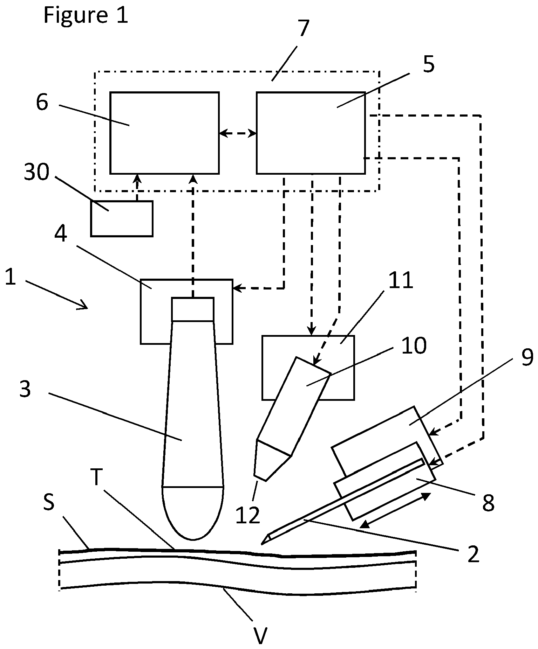

Figure 1 shows a cannula insertion system, generally denoted by reference numeral 1. The cannula insertion system 1 is configured to autonomously insert a cannula 2, for example a needle into a blood vessel V of a human or animal. The cannula insertion system 1 as shown in Figure 1 is in particular configured to autonomously draw blood from a blood vessel. In alternative embodiments, the cannula insertion system 1 may be configured to arrange a cannula in a blood vessel V for venous medication and/or infusions.

To draw blood from the blood vessel V, the cannula insertion system 1 may be arranged to determine a location of a blood vessel underneath the skin S of the human or animal, insert a cannula 2 into the blood vessel V and draw blood from the blood vessel V without direct interaction of an operator of the cannula insertion system 1.

Autonomous cannula insertion systems are known in the prior art. For example, US2008/0275396 A1 discloses an automated cannula insertion system for autonomously inserting a cannula into a blood vessel of a human or an animal, the contents of which are herein incorporated by reference in its entirety.

The cannula insertion system 1, shown on Figure 1, comprises an ultrasound transducer 3 to obtain one or more sensor signals that are representative for the location of a blood vessel V in the human or animal. The ultrasound transducer 3 is a contact sensor. During use contact between the ultrasound transducer 3 and the skin S is required in order to obtain relevant data with respect to the location of the blood vessel V. An ultrasound transducer positioning system 4 is provided to guide the ultrasound transducer along the skin S of the human or animal in a target area T. This target area T is an area of the skin S underneath which the presence of a blood vessel V suitable for insertion of a cannula 2 is expected and which is examined by the ultrasound transducer 3. A control device 5 controls the position of the ultrasound transducer 3 by sending a control signal to the ultrasound transducer positioning system 4.

The target area T may be determined by obtaining images of the skin of a human or animal, for example using a NIR (near infrared) sensor 30, and determining on the basis of the images an area in which it is likely that a blood vessel suitable for insertion of a cannula will be found.

The ultrasound transducer 3 provides a sensor signal representative for a location of the blood vessel V. The sensor signal is fed into a processing device 6 which is arranged to process the sensor signal. The control device 5 and the processing device 6 may be comprised in a single processer 7, such as a PC.

In practice, multiple sensors may be used to collect data relevant for the determination of the location of a blood vessel V underneath the skin S of a human or animal. These sensors may include contact sensors, that need to be placed on the skin S to obtain relevant data, and non-contact sensors, that can obtain information at a distance from the skin S.

On the basis of the sensor signal, or sensor signals, the processing device 6 determines the location of a blood vessel V suitable for the insertion of the cannula 2. On the basis of this location, the processing device 6 may determine an insertion path for insertion of the cannula 2 into the blood vessel V.

The cannula 2 is arranged on a cannula insertion device 8. The cannula insertion device 8 is arranged to insert the cannula 2 through the skin S and into the blood vessel V along the insertion path determined by the processing device 6. The cannula insertion device 8 is supported by a positioning system 9 that is arranged to bring the cannula insertion device 8 in a position from which the cannula insertion device 8 may move the cannula 2 along the insertion path. The cannula insertion device 8 and the positioning system 9 are controlled by the control device 5.

It is remarked that in alternative embodiments, the cannula insertion device 8 and the position system 9 may be integrated in a single cannula positioning device.

Once the cannula 2 is inserted into the blood vessel V, the cannula insertion system 1 is arranged to draw blood from the human or animal by filling one or more blood collection containers with blood. The cannula insertion system may comprise a blood container handling system to fill and close these blood collection containers. When sufficient blood has been drawn from the human or animal, the cannula insertion device 8 may withdraw the cannula 2 from the blood vessel V and out of the skin S. Thereafter, the positioning system 9 may move the cannula insertion device 8 to a location where there is no or substantially less risk that the cannula 2 will penetrate the skin S, when, for example, the body part in which the blood vessel V is present, such as an arm is withdrawn from a cannula insertion position in the cannula insertion system 1.

As indicated above, the ultrasound transducer 3 is a contact sensor. This means that in order to obtain proper data to determine a location of a blood vessel, the ultrasound transducer has to be brought into contact with the skin S. Usually a coupler medium, in particular ultrasound gel, is applied to the skin S of the target area T before the placement of the ultrasound transducer 3 on the skin S. This coupler medium improves substantially the transmission of ultrasound waves between the body of the human or animal and the ultrasound transducer 3. The application of such coupler medium, in particular ultrasound gel, is known in the art. This known ultrasound gel is manually applied on the skin before the ultrasound transducer is placed on the skin.

Thus, in a prior art automated cannula insertion system a human interaction, the manual application of ultrasound gel on the skin of the human or animal and/or on the ultrasound transducer is required; for example, the ultrasound gel may manually be applied on the ultrasound transducer in the form of a gel liquid or a gel pad.

The cannula insertion system 1 as shown in Figure 1 comprises a spray device 10 arranged to spray a coupler liquid on the skin S of the target area T. The spray device 10 can be positioned in a suitable position by a spray device positioning system 11 controlled by the control device 5. The activation of the spray device 10 to spray the coupler liquid is also controlled by the control device 5. By using a spray device 10 to apply coupler liquid to the skin S, the coupler medium will only be applied after the human or animal has placed the respective body part in a cannula insertion location of the cannula insertion system 1.

The spray device 10, or at least its spray nozzle 12, may be positioned by the spray device positioning system 11 to spray the coupler liquid on the target area T of the skin S. Furthermore, since the activation of the spray device 10, as well as the positioning of the spray device 10 is controlled by the control device 5, there is no human interaction needed to provide a coupler liquid on the target area T of the skin S. As a result, the cannula insertion system 1 can autonomously perform the step of spraying coupler liquid on the skin S within the target area. This obviates the human interaction needed in known embodiments of automated cannula insertion systems to apply ultrasound gel on the target area T of the skin S.

The spray device 10 that is controlled by the control device 5 has the further advantage that, when needed, the spray device 10 can apply further coupler liquid after measurement with the ultrasound transducer 3 has been started. In some circumstances, it may be required that additional coupler medium is applied during measurements with the ultrasound transducer 3. The cannula insertion system 1 may be arranged to apply, when desired, additional coupler liquid, for example when the measurements take longer than a predetermined period, or when it is concluded from measurements that there is insufficient coupler liquid present on the skin S of the target area T. This additional coupler liquid may be applied by the spray device 10 autonomously, without any direct human interaction being required.

The coupler liquid may be any liquid that acts as a coupler medium for the ultrasound transducer and that can be sprayed by the spray device 10. The coupler liquid may be an alcohol based liquid, a sprayable ultrasound gel, or any other sprayable coupler medium.

It is advantageous that the coupler liquid is an antiseptic liquid. By using an antiseptic coupler liquid, the coupler liquid can be used both as a coupler medium and a disinfectant for the target area T of the skin S. The application of an antiseptic coupler liquid will disinfect the target area T just before the cannula 2 will be inserted through the skin S.

The antiseptic coupler liquid may for example be an alcohol based liquid that will evaporate relatively quickly after it has been sprayed onto the skin S of the target area T. This has the advantage that the coupler medium will substantially be evaporated when the body part will be taken out of the cannula insertion location of the cannula insertion system 1. This reduces the risk of contamination of the cannula insertion system 1. Any other liquid that functions as a coupler medium and which evaporates relatively quickly may also be applied.

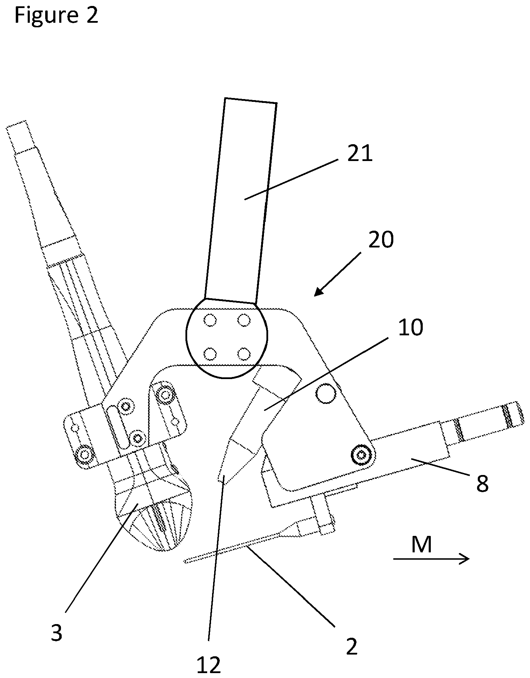

Figure 2 shows an embodiment of a measurement and cannula insertion unit 20 in which the ultrasound transducer 3, the cannula insertion device 8 and the spray device 10, or at least the spray nozzle 12 thereof, are arranged in single unit. The advantage of such single measurement and cannula insertion unit 20 is that one positioning system has to be provided for positioning the ultrasound transducer 3, the cannula insertion device 8 and the spray device 10 in a desired position. Further, since that the ultrasound transducer 3, the cannula insertion device 8 and the spray device 10 are integrated in a single unit, it is prevented that the ultrasound transducer 3, the cannula insertion device 8 and the spray device 10 may collide during movements thereof.

It is remarked that the cannula insertion device 8 is configured to move the needle 2 in a linear movement between a retracted position and an extended position in order to move the needle 2 along the insertion path. In Figure 2, the needle 2 is shown in the extended position in which the needle 2 will be pierced through the skin S when the ultrasound transducer 3 is positioned on the surface of the skin S. It will be clear that during measurements with the ultrasound transducer 3, the needle insertion device 8 will hold the needle 2 in the retracted position (translated to the right hand side in Figure 2) to enable the ultrasound transducer to be placed on the skin S without the needle 2 contacting the skin S.

The positioning system of the measurement and cannula insertion unit 20 shown in Figure 2 comprises a robot arm 21. This robot arm 21 thus acts as the ultrasound transducer positioning system 4, the positioning system 9 and the spray device positioning system 11. The spray nozzle 12 is arranged at a suitable location to spray coupler liquid on the target area T of the skin S without the need to reposition the ultrasound transducer 3 to a large extent. For example, when the measurement and cannula insertion unit 20 is moved in the direction M while the ultrasound transducer is positioned on the skin S, the spray device 10 may spray coupler liquid on a part of the skin S just before the ultrasound transducer 3 moves over this part of the skin S.

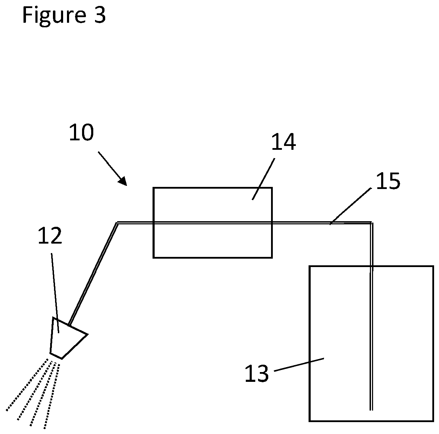

Figure 3 shows the spray device 10 in more detail. The spray device 10 comprises a spray nozzle 12, a container 13 and a pump 14. The spray nozzle 12 is in fluid communication with the container by a coupler liquid conduit 15. The coupler liquid conduit 15 runs through the pump 14. The pump 14 is connected to the control device 5. When the delivery of coupler liquid is desired the control device 5 will, when the spray nozzle 12 is positioned in the desired position, activate the pump 14 to pump coupler liquid from the container 13 to the spray nozzle 12 from which the coupler liquid will be sprayed onto the target area T.

The pump 14 may be any pump capable of pumping the coupler liquid from the container to the spray nozzle 12 with sufficient pressure to form a spray jetting from the spray nozzle 12. The pump 14 is for example a piston pump.

The container 13 is preferably a replaceable container. This replaceable container can be replaces by another container when the coupler liquid in the first container is depleted. In another embodiment, the container 13 may be refillable.

The spray nozzle 12 is movably mounted in the cannula insertion system 1 to enable positioning of the spray nozzle 12 with respect to the target area T on the skin S of the human or animal. The spray device positioning system 11 is configured to move the spray nozzle 12 to the desired location. The container 13 and the pump 14 may also be supported by the spray device positioning system 11 and move together with the spray nozzle 12. In an alternative embodiment, the container 13 and/or the pump 14 may be mounted on a fixed position in the cannula insertion system 1. This has the advantage that only the relatively light spray nozzle 12 has to be supported by the spray device positioning system 11. A flexible coupler liquid conduit may be provided to the movable spray nozzle 12 to provide the spray nozzle 12 with coupler liquid.

## Claims

1. A cannula insertion system (1) for inserting a cannula (2) into a blood vessel of a human or animal, comprising:
one or more sensors (3) to provide one or more sensor signals representative for a location of the blood vessel,
a processing device (6) to determine a location of the blood vessel on the basis of the one or more sensor signals,
a cannula insertion device (8) to insert the cannula into the blood vessel,
a positioning system (9) supporting the cannula insertion device, and
a control device (5) arranged to control the positioning system to position, on the basis of the determined location of the blood vessel, the cannula insertion device in a suitable position to insert the cannula into the blood vessel,
**characterized in that**
the one or more sensors (3) comprise an ultrasound transducer (3) to be placed on a target area of the skin of the human or animal to enable measurement with said ultrasound transducer, and **in that**
the cannula insertion system (1) comprises a coupler liquid application device (10) to apply before and/or during measuring with the ultrasound transducer coupler liquid on the target area of the skin of the human or animal, wherein the coupler liquid is used as contact liquid between the ultrasound transducer and the skin to improve measurement accuracy of the ultrasound transducer, wherein the coupler liquid application device is operated by the cannula insertion system, and wherein the coupler liquid application device is a spray device arranged to spray coupler liquid on the target area.

2. The cannula insertion system (1) of claim 1, wherein the coupler liquid is an alcohol based liquid and/or the coupler liquid is an antiseptic liquid.

3. The cannula insertion system (1) of claim 1 or 2, wherein the coupler liquid is a sprayable ultrasound gel.

4. The cannula insertion system (1) of any of the preceding claims, wherein the coupler liquid application device (10) comprises a container (13), for example a replaceable container, to hold the coupler liquid and a spray nozzle (12) in fluid communication with the container, wherein the spray nozzle is positioned to spray coupler liquid onto the target area.

5. The cannula insertion system (1) of claim 4, wherein at least the spray nozzle (12) is movable by the positioning system (9).

6. The cannula insertion system of any of the preceding claims, wherein the coupler liquid application device comprises a pump to apply coupler liquid on the target area, wherein the pump is operated by the control device.

7. The cannula insertion system (1) of any of the preceding claims, wherein the ultrasound transducer (3) is movable, and movements of the ultrasound transducer are controlled by the control device (5).

8. The cannula insertion system (1) of any of the preceding claims, wherein the cannula insertion device (8), the ultrasound transducer (3) and the coupler liquid application device (10) are arranged in a measurement and cannula insertion unit, wherein the measurement and cannula insertion unit is movable by the positioning system (9) under control of the control device (5).

9. The cannula insertion system (1) of any of the preceding claims, wherein the control device (5) and the processing device (6) are integrated in a single processing unit (7), for example a PC.

10. The cannula insertion system (1) of any of the preceding claims, wherein the cannula insertion system is an autonomous cannula insertion system constructed to autonomously insert the cannula into the blood vessel.

11. The cannula insertion system (1) of any of the preceding claims, wherein the positioning system (9) comprises a robot arm.

12. The cannula insertion system (1) of any of the preceding claims, wherein the cannula is a needle.

13. The cannula insertion system (1) of any of the preceding claims, wherein the cannula insertion system is constructed to autonomously draw blood from the blood vessel.

14. A method for determining a location of a blood vessel of a human or animal, using the cannula insertion system (1) of any of the claims 1-13, the method comprising the steps of
applying, using the coupler liquid application device (10), before and/or during measuring with the ultrasound transducer (3), coupler liquid onto the target area of the skin of the human or animal, wherein applying the coupler liquid comprises spraying the coupler liquid with the spray device,
measuring with the ultrasound transducer to provide one or more sensor signals representative for a location of the blood vessel, and
determining a location of a blood vessel on the basis of the one or more sensor signals.

15. The method of claim 14, wherein the method comprises the step of determining with one or more sensors the target area.

## Patentansprüche

1. Kanüleneinführsystem (1) zum Einführen einer Kanüle (2) in ein Blutgefäß eines Menschen oder eines Tiers, umfassend:
einen oder mehrere Sensoren (3) zum Bereitstellen eines oder mehrerer Sensorsignale, die für eine Position des Blutgefäßes repräsentativ sind,
eine Verarbeitungsvorrichtung (6) zum Bestimmen einer Position des Blutgefäßes auf der Basis des einen oder der mehreren Sensorsignale,
eine Kanüleneinführvorrichtung (8) zum Einführen der Kanüle in das Blutgefäß,
ein Positionierungssystem (9), das die Kanüleneinführvorrichtung trägt, und
eine Steuervorrichtung (5), die dazu ausgelegt ist, das Positionierungssystem so zu steuern, dass es die Kanüleneinführvorrichtung auf der Basis der bestimmten Position des Blutgefäßes in einer geeigneten Position positioniert, um die Kanüle in das Blutgefäß einzuführen,
**dadurch gekennzeichnet, dass**
der eine oder die mehreren Sensoren (3) einen Ultraschallwandler (3) umfassen, der auf einem Zielbereich der Haut des Menschen oder des Tiers zu platzieren ist, um Messung mit dem Ultraschallwandler zu ermöglichen, und dadurch, dass
das Kanüleneinführsystem (1) eine Kopplerflüssigkeitsauftragsvorrichtung (10) zum Auftragen von Kopplerflüssigkeit auf den Zielbereich der Haut des Menschen oder des Tiers vor und/oder während des Messens mit dem Ultraschallwandler umfasst, wobei die Kopplerflüssigkeit als Kontaktflüssigkeit zwischen dem Ultraschallwandler und der Haut verwendet wird, um die Messgenauigkeit des Ultraschallwandlers zu verbessern,
wobei die Kopplerflüssigkeitsauftragsvorrichtung durch das Kanüleneinführsystem betätigt wird und wobei die Kopplerflüssigkeitsauftragsvorrichtung eine Sprühvorrichtung ist, die zum Sprühen von Kopplerflüssigkeit auf den Zielbereich ausgelegt ist.

2. Kanüleneinführsystem (1) nach Anspruch 1, wobei die Kopplerflüssigkeit eine Flüssigkeit auf Alkoholbasis ist und/oder die Kopplerflüssigkeit eine antiseptische Flüssigkeit ist.

3. Kanüleneinführsystem (1) nach Anspruch 1 oder 2, wobei die Kopplerflüssigkeit ein sprühbares Ultraschallgel ist.

4. Kanüleneinführsystem (1) nach einem der vorhergehenden Ansprüche, wobei die Kopplerflüssigkeitsauftragsvorrichtung (10) einen Behälter (13), zum Beispiel einen austauschbaren Behälter, zur Aufnahme der Kopplerflüssigkeit und eine Sprühdüse (12) in Fluidverbindung mit dem Behälter umfasst, wobei die Sprühdüse zum Sprühen von Kopplerflüssigkeit auf den Zielbereich positioniert ist.

5. Kanüleneinführsystem (1) nach Anspruch 4, wobei zumindest die Sprühdüse (12) durch die Positionierungssystem (9) bewegt werden kann.

6. Kanüleneinführsystem (1) nach einem der vorhergehenden Ansprüche, wobei die Kopplerflüssigkeitsauftragsvorrichtung eine Pumpe zum Auftragen der Kopplerflüssigkeit auf den Zielbereich umfasst, wobei die Pumpe von der Steuervorrichtung betätigt wird.

7. Kanüleneinführsystem (1) nach einem der vorhergehenden Ansprüche, wobei der Ultraschallwandler (3) beweglich ist und Bewegungen des Ultraschallwandlers von der Steuervorrichtung (5) gesteuert werden.

8. Kanüleneinführsystem (1) nach einem der vorhergehenden Ansprüche, wobei die Kanüleneinführvorrichtung (8), der Ultraschallwandler (3) und die Kopplerflüssigkeitsauftragsvorrichtung in einer Mess- und Kanüleneinführeinheit angeordnet sind, wobei die Mess- und Kanüleneinführeinheit durch das Positionierungssystem (9) unter der Kontrolle der Steuervorrichtung (5) bewegt werden kann.

9. Kanüleneinführsystem (1) nach einem der vorhergehenden Ansprüche, wobei die Steuervorrichtung (5) und die Verarbeitungsvorrichtung (6) in eine einzige Verarbeitungseinheit (7), beispielsweise einen PC, integriert sind.

10. Kanüleneinführsystem (1) nach einem der vorhergehenden Ansprüche, wobei das Kanüleneinführsystem ein autonomes Kanüleneinführsystem ist, das zum autonomen Einführen der Kanüle in das Blutgefäß ausgebildet ist.

11. Kanüleneinführsystem (1) nach einem der vorhergehenden Ansprüche, wobei das Positionierungssystem (9) einen Roboterarm umfasst.

12. Kanüleneinführsystem (1) nach einem der vorhergehenden Ansprüche, wobei die Kanüle eine Nadel ist.

13. Kanüleneinführsystem (1) nach einem der vorhergehenden Ansprüche, wobei das Kanüleneinführsystem zum autonomen Entnehmen von Blut aus dem Blutgefäß ausgebildet ist.

14. Verfahren zur Bestimmung einer Position eines Blutgefäßes eines Menschen oder eines Tiers unter Verwendung des Kanüleneinführsystems (1) nach einem der Ansprüche 1 bis 13, wobei das Verfahren die folgenden Schritte umfasst:
Auftragen von Kopplerflüssigkeit auf den Zielbereich der Haut des Menschen oder des Tiers unter Verwendung der Kopplerflüssigkeitsauftragsvorrichtung (10) vor und/oder während des Messens mit dem Ultraschallwandler (3), wobei das Auftragen der Kopplerflüssigkeit ein Sprühen der Kopplerflüssigkeit mit der Sprühvorrichtung umfasst,
Messen mit dem Ultraschallwandler, um ein oder mehrere Sensorsignale bereitzustellen, die für eine Position des Blutgefäßes repräsentativ sind, und
Bestimmen einer Position eines Blutgefäßes auf der Basis des einen oder der mehreren Sensorsignale.

15. Verfahren nach Anspruch 14, wobei das Verfahren den Schritt des Bestimmens des Zielbereichs mit einem oder mehreren Sensoren umfasst.

## Revendications

1. Système d'insertion de canule (1) pour insérer une canule (2) dans un vaisseau sanguin d'un humain ou d'un animal, comprenant :
un ou plusieurs capteurs (3) pour fournir un ou plusieurs signaux de capteur représentatifs de l'emplacement du vaisseau sanguin,
un dispositif de traitement (6) pour déterminer un emplacement du vaisseau sanguin sur la base du ou des signaux de capteur,
un dispositif d'insertion de canule (8) pour insérer la canule dans le vaisseau sanguin,
un système de positionnement (9) supportant le dispositif d'insertion de canule, et
un dispositif de commande (5) agencé pour commander le système de positionnement afin de positionner, sur la base de l'emplacement déterminé du vaisseau sanguin, le dispositif d'insertion de canule dans une position appropriée pour insérer la canule dans le vaisseau sanguin,
**caractérisé en ce que**
le ou les capteurs (3) comprennent un transducteur à ultrasons (3) à placer sur une zone cible de la peau de l'homme ou de l'animal pour permettre une mesure avec ledit transducteur à ultrasons, et **en ce que**
le système d'insertion de canule (1) comprend un dispositif d'application de liquide de couplage (10) pour appliquer avant et/ou pendant la mesure avec le transducteur à ultrasons un liquide de couplage sur la zone cible de la peau de l'homme ou de l'animal, le liquide de couplage étant utilisé comme liquide de contact entre le transducteur à ultrasons, et la peau pour améliorer la précision de mesure du transducteur à ultrasons,
le dispositif d'application de liquide de couplage étant actionné par le système d'insertion de canule, et le dispositif d'application de liquide de couplage étant un dispositif de pulvérisation agencé pour pulvériser le liquide de couplage sur la zone cible.

2. Système d'insertion de canule (1) selon la revendication 1, le liquide de couplage étant un liquide à base d'alcool et/ou le liquide de couplage étant un liquide antiseptique.

3. Système d'insertion de canule (1) selon la revendication 1 ou 2, le liquide de couplage étant un gel ultrasonore pulvérisable.

4. Système d'insertion de canule (1) selon l'une quelconque des revendications précédentes, le dispositif d'application de liquide de couplage (10) comprenant un récipient (13), par exemple un récipient remplaçable, pour contenir le liquide de couplage et une buse de pulvérisation (12) en communication fluidique avec le récipient, la buse de pulvérisation étant positionnée pour pulvériser du liquide de couplage sur la zone cible.

5. Système d'insertion de canules (1) selon la revendication 4, au moins la buse de pulvérisation (12) étant déplacée par le système de positionnement (9).

6. Système d'insertion de canule selon l'une quelconque des revendications précédentes, le dispositif d'application de liquide de couplage comprenant une pompe pour appliquer du liquide de couplage sur la zone cible, la pompe étant actionnée par le dispositif de commande.

7. Système d'insertion de canule (1) selon l'une quelconque des revendications précédentes, le transducteur à ultrasons (3) étant mobile, et les mouvements du transducteur à ultrasons étant commandés par le dispositif de commande (5).

8. Système d'insertion de canule (1) selon l'une quelconque des revendications précédentes, le dispositif d'insertion de canule (8), le transducteur à ultrasons (3) et le dispositif d'application de liquide de couplage (10) étant agencés dans une unité de mesure et d'insertion de canule, l'unité de mesure et d'insertion de canule étant déplaçable par le système de positionnement (9) sous la commande du dispositif de commande (5).

9. Système d'insertion de canule (1) selon l'une quelconque des revendications précédentes, le dispositif de commande (5) et le dispositif de traitement (6) étant intégrés dans une seule unité de traitement (7), par exemple un PC.

10. Système d'insertion de canule (1) selon l'une quelconque des revendications précédentes, le système d'insertion de canule étant un système d'insertion de canule autonome conçu pour insérer de manière autonome la canule dans le vaisseau sanguin.

11. Système d'insertion de canule (1) selon l'une quelconque des revendications précédentes, le système de positionnement (9) comprenant un bras robotique.

12. Système d'insertion de canule (1) selon l'une quelconque des revendications précédentes, la canule étant une aiguille.

13. Système d'insertion de canule (1) selon l'une quelconque des revendications précédentes, le système d'insertion de canule étant conçu pour prélever le sang du vaisseau sanguin de manière autonome.

14. Procédé pour déterminer un emplacement d'un vaisseau sanguin d'un humain ou d'un animal, en utilisant le système d'insertion de canule (1) selon l'une quelconque des revendications 1 à 13, le procédé comprenant les étapes d'application, à l'aide du dispositif d'application de liquide de couplage (10), avant et/ou pendant la mesure avec le transducteur à ultrasons (3), du liquide de couplage sur la zone cible de la peau de l'humain ou de l'animal, l'application du liquide de couplage comprenant la pulvérisation du liquide de couplage à l'aide du dispositif de pulvérisation, la mesure à l'aide du transducteur à ultrasons pour fournir un ou plusieurs signaux de capteur représentatifs d'un emplacement du vaisseau sanguin, et la détermination d'un emplacement d'un vaisseau sanguin sur la base du ou des signaux de capteur.

15. Procédé selon la revendication 14, le procédé comprenant l'étape de détermination avec un ou plusieurs capteurs de la zone cible.
